# EUROPEAN PATENT APPLICATION

(11) **EP 4 834 195 A1**
(43) Date of publication of application: **04.11.2026**
(21) Application number: 24912238.3
(22) Date of filing: 03.12.2024
(51) Int. Cl.: A61B 1/00

(54) **MEDICAL OBSERVATION SYSTEM, FILTER, AND OPTICAL VIEWING TUBE**

(30) Priority: 28.12.2023 JP 2023223325
(71) Applicant: Sony Olympus Medical Solutions Inc., Hachioji-shi, Tokyo 192-0904 (JP)
(72) Inventor: DEGUCHI, Tatsuya, Hachioji-shi, Tokyo 192-0904 (JP); KOYANAGI, Masakazu, Hachioji-shi, Tokyo 192-0904 (JP)
(74) Representative: D Young & Co LLP
(86) International application number: PCT/JP2024/042769
(87) International publication number: WO 2025/142348

(57) **Abstract**

A medical observation system 1 includes: a light source device 3 that emits first excitation light; an imaging element 512 that images first fluorescent light emitted from a substance contained in an observation target OB by applying the first excitation light; and a filter 11 that is disposed on an observation optical path P1 through which the first fluorescent light propagates from the observation target OB to the imaging element 512 and that inhibits light in a first wavelength band, which is at least a part of a wavelength band of the first fluorescent light, so that a first output signal value from the imaging element 512 that has received the first fluorescent light approaches a predetermined signal value.

## Description

### Field

The present disclosure relates to a medical observation system, a filter, and an optical visual tube. Background

A medical observation system has been known in which excitation light is applied to an observation target (subject such as human) and fluorescent light (hereinafter, referred to as observation target fluorescent light) emitted from a substance contained in the observation target by applying the excitation light is observed (e.g., see Patent Literature 1). The excitation light is narrowband light emitted from a light source device.

According to such fluorescent light observation, a tissue state difficult to be recognized can be grasped via observation target fluorescent light. Therefore, fluorescent light observation can be used for various purposes and applications such as identification of a lesion portion.

### Citation List

### Patent Literature

Patent Literature 1: JP 2021-132695 A

### Summary

### Technical Problem

Then, improving performance in fluorescent light observation is desired.

The present disclosure has been made in view of the above, and an object thereof is to provide a medical observation system, a filter, and an optical visual tube capable of improving performance in fluorescent light observation.

### Solution to Problem

To solve the above-described problem and achieve the object, a medical observation system according to the present disclosure includes: a light source device configured to emit first excitation light; an imaging element configured to image first fluorescent light emitted from a substance contained in an observation target by applying the first excitation light; and a filter disposed on an observation optical path through which the first fluorescent light propagates from the observation target to the imaging element and configured to inhibit light in a first wavelength band, which is at least a part of a wavelength band of the first fluorescent light, such that a first output signal value from the imaging element that has received the first fluorescent light approaches a predetermined signal value.

Moreover, a filter according to the present disclosure is disposed on an observation optical path through which the first fluorescent light propagates from an observation target to which first excitation light is applied to an imaging element configured to image first fluorescent light emitted from a substance contained in the observation target by applying the first excitation light and is configured to inhibit light in a first wavelength band, which is at least a part of a wavelength band of the first fluorescent light, such that a first output signal value from the imaging element that has received the first fluorescent light approaches a predetermined signal value.

Moreover, an optical visual tube according to the present disclosure applies first excitation light to an observation target and captures first fluorescent light emitted from a substance contained in the observation target by applying the first excitation light, the optical visual tube including a filter disposed on an observation optical path through which the first fluorescent light propagates and configured to inhibit light in a first wavelength band, which is at least a part of a wavelength band of the first fluorescent light, such that a first output signal value from an imaging element that has received the first fluorescent light approaches a predetermined signal value.

### Advantageous Effects of Invention

The medical observation system, the filter, and the optical visual tube according to the present disclosure can improve performance in fluorescent light observation.

### Brief Description of Drawings

FIG. 1 illustrates a configuration of a medical observation system according to an embodiment.
FIG. 2 illustrates the function of a filter.
FIG. 3 illustrates effects of the embodiment.
FIG. 4 illustrates Variation 1 of the embodiment.
FIG. 5 illustrates Variation 2 of the embodiment.
FIG. 6 illustrates Variation 3 of the embodiment.
FIG. 7 illustrates Variation 4 of the embodiment.
FIG. 8 illustrates Variation 4 of the embodiment.
FIG. 9 illustrates Variation 5 of the embodiment.
FIG. 10 illustrates Variation 6 of the embodiment.
FIG. 11 illustrates Variation 7 of the embodiment.
FIG. 12 illustrates Variation 8 of the embodiment.
FIG. 13 illustrates Variation 9 of the embodiment.
FIG. 14 illustrates Variation 10 of the embodiment.
FIG. 15 illustrates Variation 11 of the embodiment.
FIG. 16 illustrates Variation 12 of the embodiment.
FIG. 17 illustrates Variation 12 of the embodiment.

### Description of Embodiments

An embodiment for carrying out the present disclosure (hereinafter, embodiment) will be described below with reference to the drawings. Note that the present disclosure is not limited by the embodiment to be described below. Moreover, in the drawings, the same reference signs are attached to the same parts.

### [Configuration of Medical Observation System]

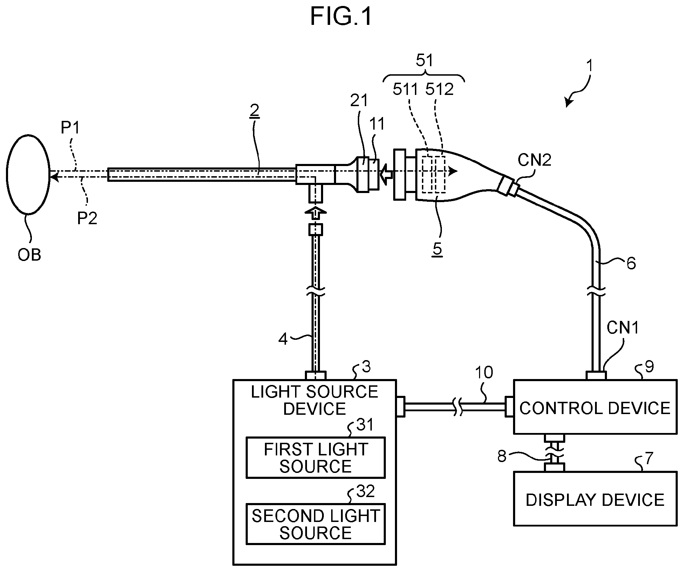

FIG. 1 illustrates a configuration of a medical observation system 1 according to the embodiment.

In the embodiment, the medical observation system 1 is a medical endoscope system for observing an observation target OB (inside of living body) with an endoscope. As illustrated in FIG. 1, the medical observation system 1 includes an insertion portion 2, a light source device 3, a light guide 4, a camera head 5, a first transmission cable 6, a display device 7, a second transmission cable 8, a control device 9, a third transmission cable 10, and a filter 11.

In the embodiment, the insertion portion 2 includes a rigid endoscope, and corresponds to an optical visual tube according to the present disclosure. That is, the insertion portion 2 has an elongated shape. The insertion portion 2 is entirely rigid, or has a flexible part and the other rigid part. The insertion portion 2 is to be inserted into the observation target OB. An optical system is provided in the insertion portion 2. The optical system includes one or a plurality of lenses, and collects return light (subject image) from the observation target OB.

One end of the light guide 4 is connected to the light source device 3. Then, the light source device 3 includes a first light source 31 (FIG. 1) and a second light source 32 (FIG. 1). The first light source 31 supplies first excitation light to one end of the light guide 4 under the control of the control device 9. The second light source 32 supplies second excitation light to one end of the light guide 4. Note that the first excitation light may be visible light or invisible light. Similarly, the second excitation light may be visible light or invisible light. Furthermore, the first light source 31 may include a light emitting diode (LED) or a semiconductor laser. Similarly, the second light source 32 may include an LED or a semiconductor laser. Furthermore, one or a plurality of first light sources 31, which emit the first excitation light, may be provided. Similarly, one or a plurality of second light sources 32, which emit the second excitation light, may be provided.

Here, examples of substances contained in the observation target OB to be excited by the first and second beams of excitation light can include medical agents and fluorescent dyes imparted to the observation target OB and fluorescent substances derived from the observation target OB constituting the observation target OB itself.

Examples of the above-described medical agents to be imparted to the observation target OB can include "5-ALA (PP-IX)", "ADS780WS", "ADS830WS", "aggregation-induced emission dots allophycocyanin (APC)", "boron-dipyrromethane (BODIPY)", "CLR 1502", "Flavins", "fluorescamine", "Fluorescein", "fluoro-gold", "green fluorescence protein", "indocyanine green (ICG)", "IRDye 78", "IR-PEG nanoparticles", "Isothiocyanate", "rose Bengal", "SGM-101", and "trypan blue".

Furthermore, examples of the above-described fluorescent dyes to be imparted to the observation target OB can include "coumarine", "Cy3", "DyLight547", "GE3126", "metal nanoclusters", "oxacarbocyanine", "Rhodamine", "Riboflavin", "fluorescein", "AlexaFluor 488", "AlexaFluor660", "AlexaFluor680", "AlexaFluor700", "Cy5", "Cy5.5", "Dy677", "Dy682", "Dy752", "DyLight647", "HiLyte Fluor 647", "HiLyte Fluor 680", "IRDye 700DX", "methylene blue", "Porphyrins", "Porphysomes", "VivoTag-680", "VivoTag-S680", "AlexaFluor750", "AlexaFluor790", "carbocyanine", "conjugated copolymers", "CW800-CA", "Cy7", "Cy7.5", "cyanine dyes", "Dy780", "HiLyte Fluor 750", "Indocarbocyanine", "IR-786", "IRDye 800CW", "IRDye 800RS", "IRDye 800BK", "Nervelight", "OTL-38 (Pafolacianine)", "Polymethine", "VivoTag-S750", "ASP5354", "Xanthene", and "LUM-015".

Moreover, examples of the fluorescent substances derived from the observation target OB constituting the observation target OB itself can include "collagen", "elastin", and "NADH".

Note that, although the light source device 3 is separated from the control device 9 in the embodiment, this is not a limitation. A configuration provided in the same casing as the control device 9 may be adopted.

One end of the light guide 4 is detachably connected to the light source device 3. Furthermore, the other end of the light guide 4 is detachably connected to the insertion portion 2. Then, the light guide 4 propagates the first and second beams of excitation light supplied from the light source device 3 (first and second light sources 31 and 32) from one end to the other end, and supplies the first and second beams of excitation light to the insertion portion 2. Each of the first and second beams of excitation light supplied to the insertion portion 2 is emitted from a distal end of the insertion portion 2, and applied to the observation target OB. The optical system in the insertion portion 2 collects each of beams of return light (subject images) of the first and second beams of excitation light from the observation target OB. Each of the first and second beams of excitation light has been applied to the observation target OB. In addition to the first excitation light reflected by the observation target OB, the return light of the first excitation light includes fluorescent light (hereinafter, referred to as first fluorescent light), which is emitted from a substance by applying the first excitation light to the observation target OB and exciting the substance contained in the observation target OB. Furthermore, in addition to the second excitation light reflected by the observation target OB, the return light of the second excitation light includes fluorescent light (hereinafter, referred to as second fluorescent light), which is emitted from a substance by applying the second excitation light to the observation target OB and exciting the substance contained in the observation target OB.

The camera head 5 is detachably connected to a proximal end (eyepiece portion 21 (FIG. 1)) of the insertion portion 2 via the filter 11. As illustrated in FIG. 1, the camera head 5 includes an imaging unit 51.

As illustrated in FIG. 1, the imaging unit 51 includes a lens unit 511 and an imaging element 512.

The lens unit 511 forms an image of each of the beams of return light (subject images) of the first and second beams of excitation light collected by the insertion portion 2 on a light receiving surface of the imaging element 512.

The imaging element 512 includes a charge coupled device (CCD) or a complementary metal oxide semiconductor (CMOS), which receives each of the beams of return light (subject images) of the first and second beams of excitation light and converts each of the beams of return light (subject images) into an electric signal. The lens unit 511 has formed an image of each of the beams of return light (subject images).

Then, the imaging unit 51 outputs a pixel signal obtained by the imaging under the control of the control device 9.

One end CN1 of the first transmission cable 6 is detachably connected to the control device 9. Furthermore, the other end CN2 of the first transmission cable 6 is detachably connected to the camera head 5. Note that the other end CN2 is not limited to having a configuration in which the other end CN2 is detachably connected to the camera head 5. A configuration in which the other end CN2 is fixed to the camera head 5 may be adopted. Then, the first transmission cable 6 transmits the pixel signal output from the imaging unit 51 to the control device 9, and transmits, to the camera head 5, each of a control signal, a synchronization signal, a clock, power, or the like, which have been sent from the control device 9.

Note that the pixel signal or the like may be transmitted from the camera head 5 to the control device 9 via the first transmission cable 6 in a form of an optical signal or an electric signal. The control signal, the synchronization signal, and the clock may also be transmitted similarly from the control device 9 to the camera head 5 via the first transmission cable 6.

The display device 7 includes a display formed of, for example, liquid crystal or organic electro luminescence (EL). The display device 7 displays an image based on a video signal from the control device 9 under the control of the control device 9.

One end of the second transmission cable 8 is detachably connected to the display device 7. Furthermore, the other end of the second transmission cable 8 is detachably connected to the control device 9. Then, the second transmission cable 8 transmits the video signal processed by the control device 9 to the display device 7.

The control device 9 includes a controller such as a central processing unit (CPU) and a micro processing unit (MPU), and integrally controls operations of the light source device 3, the camera head 5, and the display device 7. Note that the control device 9 may include not only the CPU and the MPU but an integrated circuit such as an application specific integrated circuit (ASIC), a field-programmable gate array (FPGA), and a graphics processing unit (GPU).

Specifically, the control device 9 generates a video signal by performing various pieces of processing on a pixel signal acquired from the camera head 5 via the first transmission cable 6, and outputs the video signal to the display device 7 via the second transmission cable 8. Then, the display device 7 displays an image based on the video signal. Furthermore, the control device 9 outputs, for example, a control signal to the camera head 5 and the light source device 3 via first and third transmission cables 6 and 10.

One end of the third transmission cable 10 is detachably connected to the light source device 3. Furthermore, the other end of the third transmission cable 10 is detachably connected to the control device 9. Then, the third transmission cable 10 transmits a control signal from the control device 9 to the light source device 3.

Incidentally, in general, influence of (1) to (4) below makes it difficult to adjust a signal value based on fluorescent light (first and second beams of fluorescent light) generated by the imaging element 512 that has received the fluorescent light in the medical observation system 1.

### (1) Medical Agent

In general, a type and a dosage of a medical agent vary an amount of fluorescent light emitted from the medical agent.

The type of a medical agent to be administered to the observation target OB is selected in accordance with the observation target OB (e.g., cancer, blood, and lymph). Moreover, a medical agent capable of separating the wavelength of excitation light that excites the medical agent from the wavelength of fluorescent light emitted from the medical agent is selected in order to image the fluorescent light emitted from the medical agent in the medical observation system 1. Medical agents selected as described above emit different amounts of fluorescent light.

Furthermore, although the amount of the fluorescent light can be adjusted by the dosage of a medical agent, unnecessarily increasing the dosage is difficult for achieving minimally invasive treatment.

That is, it is difficult to adjust the amount of the fluorescent light by selecting the type of a medical agent or adjusting a dosage. As a result, it is difficult to adjust the signal value based on the fluorescent light imaged by the imaging element 512 by selecting the type of a medical agent or adjusting a dosage.

### (2) Observation Target

The amount of the fluorescent light emitted from the observation target OB varies depending on the part/state of the observation target OB.

Specifically, when the observation target OB is a part/state in which a medical agent easily stagnates, the amount of the fluorescent light emitted from the observation target OB increases. In contrast, when the observation target OB is a part/state in which a medical agent easily flows and not easily stagnates, the amount of the fluorescent light emitted from the observation target OB decreases, and afterglow time is also shortened. Furthermore, when the observation target OB is a tumor, the amount of the fluorescent light received by the imaging element 512 changes depending on the spread, size, and depth thereof.

That is, it is difficult to adjust the amount of the fluorescent light depending on the type and situation of the observation target OB. As a result, it is difficult to adjust the signal value based on the fluorescent light imaged by the imaging element 512 depending on the type and situation of the observation target OB.

### (3) Light Source Device

The amount of the excitation light emitted from the light source device 3 varies the amount of the fluorescent light.

In order to increase the amount of the excitation light, adjusting power to be supplied to the light source device 3 may be necessary. Power that can be supplied to the light source device 3 is, however, restricted in accordance with an upper limit value of power for operating the entire medical observation system 1. Furthermore, the amount of the excitation light is required to be adjusted in consideration of heat generation in a member constituting an optical path of the excitation light (e.g., heat generation between light guide 4 and insertion portion 2), conformance with a laser class, an amount of light energy received by the observation target OB or a surrounding living body (large amount of light energy may lead to burn), or speed of fading of the fluorescent light emitted from a medical agent. Moreover, the amount of the excitation light can be adjusted also by changing the number of light sources of the excitation light mounted on the light source device 3. The number of light sources, however, may influence the size of the light source device 3. The size of the light source device 3 may be restricted by the size of a cart for carrying the light source device 3 and the like.

That is, it is difficult to adjust the amount of the fluorescent light by adjusting the amount of the excitation light. As a result, it is difficult to adjust the signal value based on the fluorescent light imaged by the imaging element 512 by adjusting the amount of the excitation light.

### (4) Imaging Element

The signal value based on the fluorescent light generated by the imaging element 512 varies depending on the amount of the fluorescent light received by the imaging element 512.

In order to adjust the signal value based on the fluorescent light, the imaging element 512 having sensitivity, configuration, and the like optimal to imaging of the fluorescent light is desirably selected. The imaging element 512 may be, however, required not only to output an image for fluorescent light observation based on reception of the fluorescent light in a predetermined wavelength band but to output an image for normal light observation based on reception of visible light such as white light. Furthermore, the imaging element 512 may also be required to output an image for fluorescent light observation in a wide wavelength band or a plurality of wavelength bands among wavelength bands containing visible light and invisible light. Moreover, even when the same medical agent is used, the amount of the fluorescent light may change depending on a procedure or the observation target OB. The imaging element 512 may also be required to output an image for fluorescent light observation in accordance with such a change in the amount of the fluorescent light. In that case, the imaging element 512 needs to select an element that can address such observation. It may be impossible to use an imaging element having characteristics optimal to imaging of fluorescent light in a predetermined wavelength band. Furthermore, the imaging element 512 is disposed in the camera head 5. There are a size and a weight of the camera head 5 suitable for observation, which may restrict the type including the size of the imaging element 512. Note that not only in the configuration in which the imaging element 512 is disposed in the camera head 5 but in a case where the imaging element 512 is disposed at a distal end of a rigid endoscope or a flexible endoscope, there are a size and a weight suitable for observation, which may restrict the type including the size of the imaging element 512.

That is, it is difficult to adjust the signal value based on the fluorescent light imaged by the imaging element 512 by selecting the type of the imaging element 512.

As described above, the signal value based on the fluorescent light imaged by the imaging element 512 is determined under the above-described restrictions, so that the signal value cannot be easily adjusted.

Furthermore, first and second medical agents are assumed as medical agents to be administered to the observation target OB. The second medical agent has weak fluorescent light. The first medical agent has strong fluorescent light.

Then, for the second medical agent having poor fluorescence efficiency, the signal value based on the fluorescent light imaged by the imaging element 512 is adjusted by a combination of an approach and a parameter. The approach includes increasing an emission amount of excitation light, increasing emission time of the excitation light, prolonging exposure time of the imaging element 512, and increasing an electric gain that amplifies an output signal from the imaging element 512 by signal processing. In contrast, for the first medical agent having good fluorescence efficiency, the signal value based on the fluorescent light imaged by the imaging element 512 is adjusted by a combination of an approach and a parameter. The approach includes decreasing an emission amount of excitation light, decreasing emission time of the excitation light, shortening exposure time of the imaging element 512, and decreasing an electric gain that amplifies an output signal from the imaging element 512 by signal processing. Restrictions are, however, imposed on an approach that can be implemented by the light source device 3, the imaging element 512, and the signal processing and a parameter. There is such a problem that one device cannot necessarily implement the optimal approach and parameter to the first and second medical agents.

In the embodiment, the filter 11 is adopted to solve the above-described problem. The filter 11 is disposed on an observation optical path P1 (FIG. 1) through which the beams of return light (subject images) of the first and second beams of excitation light propagate from the observation target OB to the imaging element 512. More specifically, the filter 11 is disposed between the eyepiece portion 21 and the camera head 5 (FIG. 1). Furthermore, the filter 11 is detachably connected to the eyepiece portion 21.

The filter 11 has a configuration in which a film of an inorganic material is optically formed on, for example, synthetic quartz. The synthetic quartz is a base material that emits a small amount of auto-fluorescent light. The filter 11 has a band-pass function, a short-pass function, or a long-pass function.

Note that details of the function of the filter 11 will be described later in "Function of Filter".

### [Function of Filter]

Next, the function of the above-described filter 11 will be described.

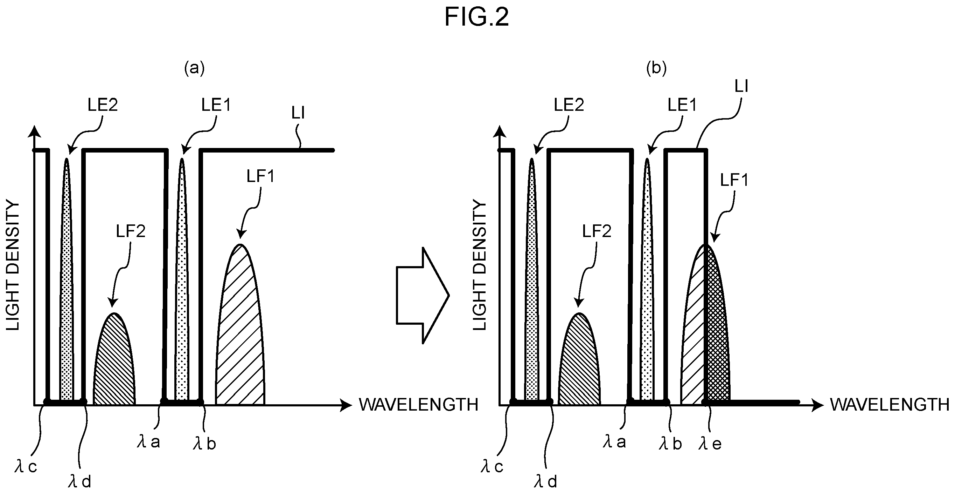

FIG. 2 illustrates the function of the filter 11. Specifically, FIG. 2 illustrates the spectra of first excitation light LE1, second excitation light LE2, first fluorescent light LF1, and second fluorescent light LF2. Then, lines LI indicate the transmission characteristics (filter characteristics) of the filter 11. Note that, in FIG. 2, the horizontal axis represents a wavelength [nm], and the vertical axis represents a light density [W/nm].

FIG. 2 illustrates a case where the first fluorescent light LF1 has a light density larger than that of the second fluorescent light LF2.

Then, in the embodiment, the filter 11 has an excitation light cut function and a fluorescent light inhibiting function. In the excitation light cut function, the first and second beams of excitation light LE1 and LE2 contained in the beams of return light (subject images) of the first and second beams of excitation light LE1 and LE2 are cut. In the fluorescent light inhibiting function, the first fluorescent light LF1 contained in the beams of return light is inhibited. Here, the "cut" means partially, substantially, or completely inhibiting light. "Cut" to be described below has similar meaning. Note that (a) of FIG. 2 illustrates a case where the filter 11 has only the excitation light cut function among the excitation light cut function and the fluorescent light inhibiting function. In contrast, (b) of FIG. 2 illustrates a case where the filter 11 has both the excitation light cut function and the fluorescent light inhibiting function.

Specifically, the excitation light cut function is as follows.

As indicated by the lines LI in FIG. 2, the filter 11 has the excitation light cut function of cutting light having wavelengths λa to λb and cutting light having wavelengths λc to λd. The wavelengths λa to λb constitute a wavelength band of the first excitation light LE1. The wavelengths λc to λd constitute a wavelength band of the second excitation light LE2.

Furthermore, the fluorescent light inhibiting function is as follows.

As indicated by a line LI in (b) of FIG. 2, the filter 11 has the fluorescent light inhibiting function of inhibiting light in a first wavelength band (wavelength λe or more), which is a part of a wavelength band of the first fluorescent light LF1.

Note that, as indicated by the line LI in (b) of FIG. 2, the filter 11 substantially or completely transmits all light outside the wavelength bands of the wavelengths λa to λb, the wavelengths λc to λd, and the wavelength λe or more.

Then, when the filter 11 does not have the fluorescent light inhibiting function ((a) of FIG. 2), a signal value of an output signal (hereinafter, referred to as first output signal S1) from the imaging element 512 that has received the first fluorescent light LF1 is larger than a signal value of an output signal (hereinafter, referred to as second output signal S2) from the imaging element 512 that has received the second fluorescent light LF2. In contrast, when the filter 11 has the fluorescent light inhibiting function ((b) of FIG. 2), the signal value of the first output signal S1 approaches the signal value of the second output signal S2. Note that the signal value of the first output signal S1 is only required to approach the signal value of the second output signal S2. The signal values may be the same as each other, and are not required to be the same as each other. That is, the filter 11 inhibits light in the first wavelength band so that the signal value of the first output signal S1 approaches a predetermined signal value (signal value of second output signal S2) by the fluorescent light inhibiting function. The first wavelength band is at least a part of the wavelength band of the first fluorescent light LF1.

According to the above-described embodiment, the following effects are exhibited.

The medical observation system 1 according to the first embodiment includes the filter 11 that is disposed on the observation optical path P1 and that inhibits light in the first wavelength band, which is at least a part of the wavelength band of the first fluorescent light LF1 so that the signal value of the first output signal S1 approaches the predetermined signal value (signal value of second output signal S2). That is, in a case where the same system performs fluorescent light observation using the first fluorescent light LF1 and fluorescent light observation using the second fluorescent light LF2, the signal values of the first and second output signals S1 and S2 can be substantially the same as each other, which can make control processing in the control device 9 simple, and improve performance in fluorescent light observation.

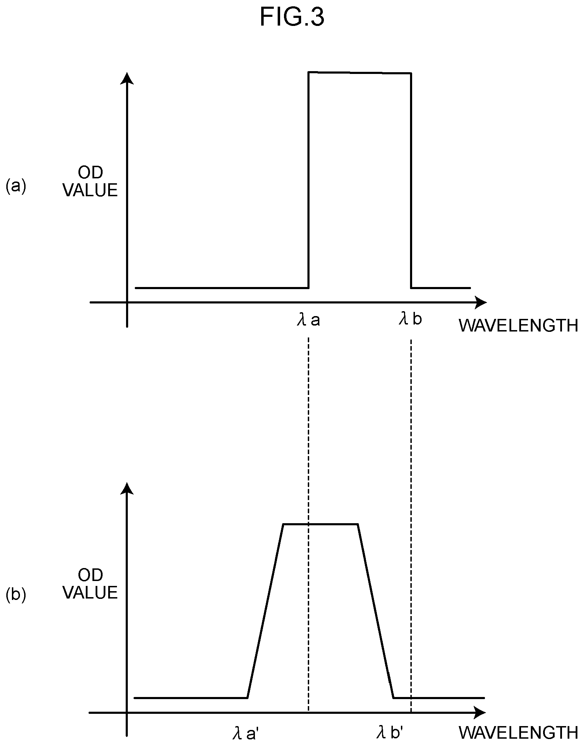

FIG. 3 illustrates effects of the embodiment. Specifically, (a) of FIG. 3 illustrates the transmission characteristics (filter characteristics) of the filter 11 in a case where the filter 11 is disposed at a specific position (between eyepiece portion 21 and camera head 5). In contrast, (b) of FIG. 3 illustrates the transmission characteristics (filter characteristics) of the filter 11 in a case where the filter 11 is disposed at a position other than the specific position. Note that, in FIG. 3, the horizontal axis represents a wavelength [nm], and the vertical axis represents an optical density (OD value). Furthermore, for convenience of description, FIG. 3 pays attention only to the wavelength band of the wavelengths λa to λb as the transmission characteristics of the filter 11.

The transmission characteristics of the filter 11 change depending on an incident angle of light.

Light emitted from the eyepiece portion 21 is substantially parallel light. Therefore, when the filter 11 is disposed between the eyepiece portion 21 and the camera head 5, the beams of return light of the first and second beams of excitation light LE1 and LE2 are incident on the filter 11 as substantially parallel beams of light. When the beams of return light of the first and second beams of excitation light LE1 and LE2 are incident as substantially parallel beams of light as described above, the filter 11 has substantially transmission characteristics of a design value as illustrated in (a) of FIG. 3.

In contrast, in a case of light beam distribution of NA 0.2 or more (light beam of ±12 degrees or more), as illustrated in (b) of FIG. 3, the filter 11 may have transmission characteristics deteriorated from the transmission characteristics of the design value. Specifically, a wavelength band in which cutting is performed shifts to the side of a short wavelength, so that the wavelength band in which cutting is performed may be no longer the optimal wavelength band. Furthermore, rise and fall of the OD value in the filter characteristics may change from sharp rise and fall ((a) of FIG. 3) in design to gentle rise and fall ((b) of FIG. 3). Moreover, the OD value of the wavelength band in which cutting is performed may decrease.

As described above, the transmission characteristics of the filter 11 can be maintained at the transmission characteristics of a design value, and the first fluorescent light LF1 can be inhibited at the design value by arranging the filter 11 between the eyepiece portion 21 and the camera head 5.

Furthermore, the filter 11 may be detachably connected to the eyepiece portion 21.

In such a detachable configuration, if a plurality of filters 11 having different wavelength bands in which cutting is desired to be performed and different levels of an amount of light desired to be cut is prepared in accordance with a medical agent and a fluorescent dye to be imparted to the observation target OB, the filter 11 can be selected and used in accordance with an observation target substance such as the medical agent and the fluorescent dye to be used.

Incidentally, when the first and second beams of excitation light LE1 and LE2 and the beams of return light of the first and second beams of excitation light LE1 and LE2 are applied to members (hereinafter, referred to as auto-fluorescent light generating members) forming optical paths P1 and P2 at the time when the first and second beams of excitation light LE1 and LE2 propagate through the optical path P2 (FIG. 1) and at the time when the beams of return light propagate through the observation optical path P1, auto-fluorescent light may be generated from the auto-fluorescent light generating members. The optical path P2 follows a path of the light source device 3 to the light guide 4 to the insertion portion 2 to the observation target OB. Examples of the auto-fluorescent light generating members can include a component contained in multicomponent glass such as a lens, a material component contained in a color filter, an adhesive that joins lenses or other optical members, and oil attached to an optical member such as a lens. Such auto-fluorescent light has a wavelength band including the wavelength bands of the beams of observation target fluorescent light (first and second beams of fluorescent light LF1 and LF2) in fluorescent light observation, and serves as noise in performing the fluorescent light observation. Note that, also when the first and second beams of excitation light LE1 and LE2 are applied to the observation target OB, such auto-fluorescent light may be generated.

In contrast, the filter 11 has the fluorescent light inhibiting function of inhibiting light having the wavelength λe or more. Therefore, auto-fluorescent light having the wavelength λe or more can be inhibited, and the fluorescent light observation can be successfully performed. The auto-fluorescent light having the wavelength λe or more is part of the above-described auto-fluorescent light, and serves as noise in performing fluorescent light observation with the filter 11.

### (Other Embodiments)

Although the embodiment for carrying out the present disclosure has been described so far, the present disclosure should not be limited only by the above-described embodiment.

In the above-described embodiment, not only one or two or more filters according to the present disclosure may be provided. For example, although, in the above-described embodiment, only one filter 11 (hybrid filter) is provided for the first and second beams of fluorescent light LF1 and LF2, a filter may be provided for each of the first and second beams of fluorescent light LF1 and LF2. The filter 11 of a hybrid type can reduce the number of filters, and reduce the size and weight of the entire system. Furthermore, the filter of the present disclosure may be disposed not only at the arrangement position described in the above-described embodiment but at any position on the observation optical path P1. The filter according to the present disclosure may be disposed in, for example, the insertion portion 2 or the camera head 5 (e.g., imaging surface of imaging element 512) as long as the filter is located on the observation optical path P1. Furthermore, when two or more filters according to the present disclosure are provided, all the filters may be disposed at the same position, or may be disposed at different positions.

In the above-described embodiment, the predetermined signal value according to the present disclosure is not limited to the signal value of the second output signal S2. For example, a signal value capable of inhibiting the fluorescence intensity of a medical agent into a range in which the imaging element 512 can perform imaging may be set as the predetermined signal value according to the present disclosure. That is, in other words, a value smaller than a signal value at which a pixel of the imaging element 512 saturates may be set as the predetermined signal value according to the present disclosure. Furthermore, although, in the above-described embodiment, a plurality of medical agents is used, only one medical agent may be used. Moreover, for example, a signal value set based on brightness-related information may be set as the predetermined signal value according to the present disclosure. The brightness-related information specifies brightness in performing fluorescent light observation. The brightness-related information includes at least one or a plurality of the type (e.g., cancer, blood, and lymph) of the observation target OB, the type of a medical agent, a surgical procedure, and a device constituting a system. That is, even when the same medical agent is used, fluorescence intensity changes depending on, for example, the type of the observation target OB and a surgical procedure. Therefore, a signal value that suits a need of performing fluorescent light observation at specific brightness may be set as the predetermined signal value according to the present disclosure. Moreover, for example, a signal value under the same medical agent and the same condition in a past model or a different model may be set as the predetermined signal value according to the present disclosure.

Variations 1 to 10 below may be adopted in the above-described embodiment.

### (Variation 1)

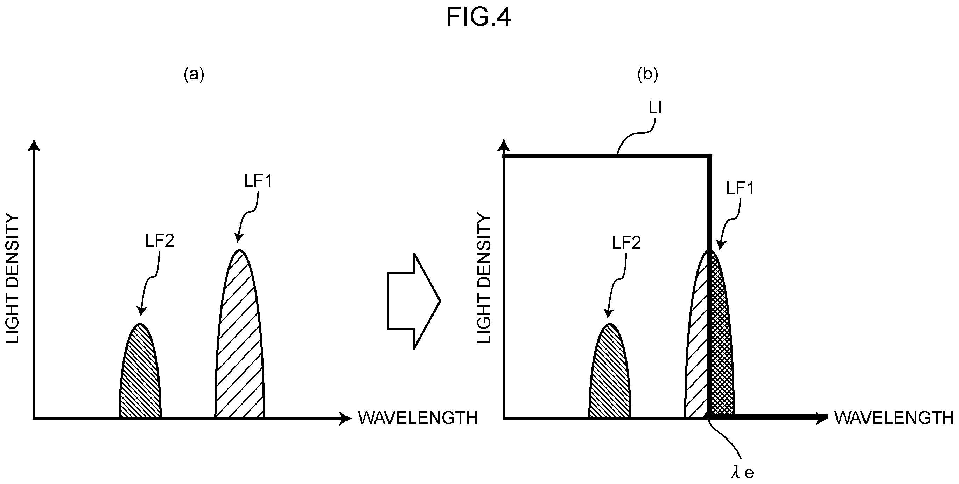

FIG. 4 illustrates Variation 1 of the embodiment. Specifically, FIG. 4 corresponds to FIG. 2. More specifically, (a) of FIG. 4 illustrates the spectra of the first and second beams of fluorescent light LF1 and LF2 immediately before the first and second beams of fluorescent light LF1 and LF2 are transmitted through the filter 11 according to Variation 1. Furthermore, (b) of FIG. 4 illustrates the spectra of the first and second beams of fluorescent light LF1 and LF2 after the first and second beams of fluorescent light LF1 and LF2 have been transmitted through the filter 11 according to Variation 1.

Although, in the above-described embodiment, the filter 11 has both the excitation light cut function and the fluorescent light inhibiting function, this is not a limitation. For example, as in the filter 11 according to Variation 1 indicated by the line LI in (b) of FIG. 4, a configuration having only the fluorescent light inhibiting function among the excitation light cut function and the fluorescent light inhibiting function may be adopted. That is, in Variation 1, a filter having the excitation light cut function is separately provided on the optical path front stage side of the filter 11 according to Variation 1.

Even when the filter 11 according to Variation 1 above is adopted, effects similar to those of the above-described embodiment are exhibited.

### (Variation 2)

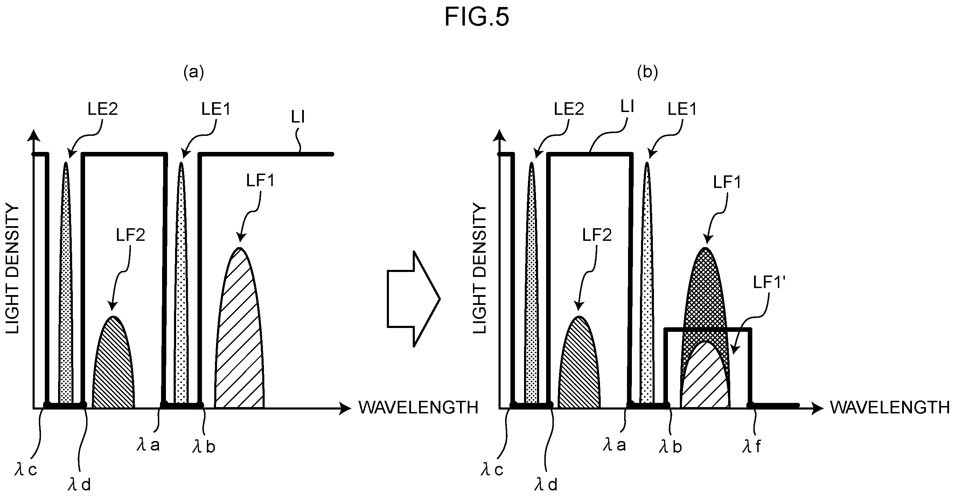

FIG. 5 illustrates Variation 2 of the embodiment. Specifically, FIG. 5 corresponds to FIG. 2.

In Variation 2, a neutral density (ND) filter is adopted as the filter 11 in the above-described embodiment. Note that an electronic ND filter that performs electric adjustment may be adopted as the filter 11. Specifically, as indicated by a line LI in (b) of FIG. 5, the filter 11 according to Variation 2 has the excitation light cut function similar to that in the above-described embodiment, and has the fluorescent light inhibiting function to be described below.

As indicated by the line LI in (b) of FIG. 5, the filter 11 according to Variation 2 has the fluorescent light inhibiting function of reducing light transmittance of a first wavelength band (wavelengths λb to λf) including all the wavelength band of the first fluorescent light LF1 and setting first fluorescent light LF1'. In the case, the hue of the first fluorescent light LF1' is maintained with respect to the first fluorescent light LF1. Note that a state in which the hue is maintained means, for example, a hue difference of approximately 5 or less in uniform color space such as L*a*b*.

Then, as in the above-described embodiment, the filter 11 according to Variation 2 makes the signal value of the first output signal S1 approach the signal value of the second output signal S2.

Even when the filter 11 according to Variation 2 above is adopted, effects similar to those of the above-described embodiment are exhibited.

### (Variation 3)

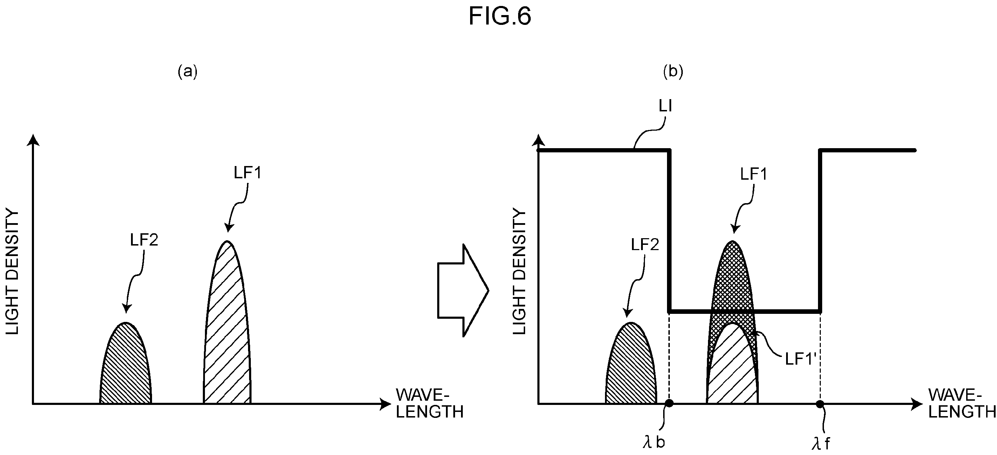

FIG. 6 illustrates Variation 3 of the embodiment. Specifically, FIG. 6 corresponds to FIG. 2. More specifically, (a) of FIG. 6 illustrates the spectra of the first and second beams of fluorescent light LF1 and LF2 immediately before the first and second beams of fluorescent light LF1 and LF2 are transmitted through the filter 11 according to Variation 3. Furthermore, (b) of FIG. 6 illustrates the spectra of the first and second beams of fluorescent light LF1 and LF2 after the first and second beams of fluorescent light LF1 and LF2 have been transmitted through the filter 11 according to Variation 3.

Although, in Variation 2 above, the filter 11 has both the excitation light cut function and the fluorescent light inhibiting function, this is not a limitation. For example, as in the filter 11 according to Variation 3 indicated by a line LI in (b) of FIG. 6, a configuration having only the fluorescent light inhibiting function among the excitation light cut function and the fluorescent light inhibiting function may be adopted. That is, in Variation 3, a filter having the excitation light cut function is separately provided on the optical path front stage side of the filter 11 according to Variation 3.

Even when the filter 11 according to Variation 3 above is adopted, effects similar to those of Variation 2 above are exhibited.

### (Variation 4)

In Variation 4, the configurations of the imaging unit 51 and the imaging element 512 and the function of the filter 11 are changed from those in the above-described embodiment. For convenience of description, the imaging unit 51, the imaging element 512, and the filter 11 according to Variation 4 will be hereinafter referred to as an imaging unit 51A, an imaging element 512A, and a filter 11A.

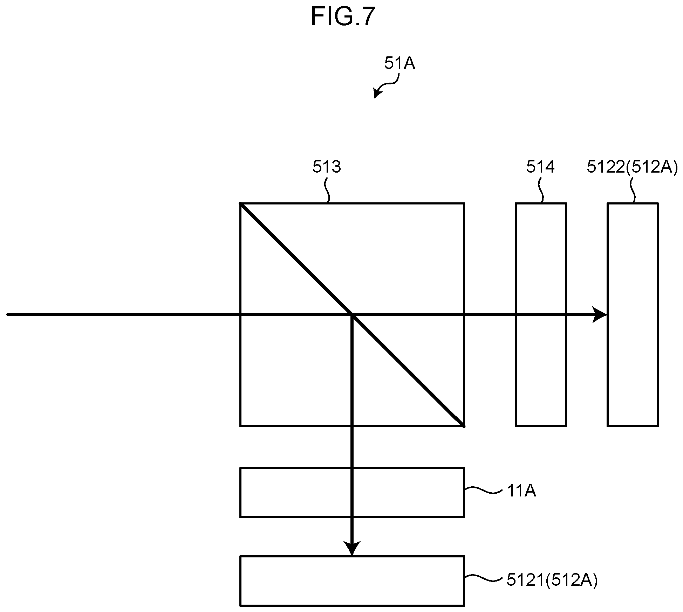

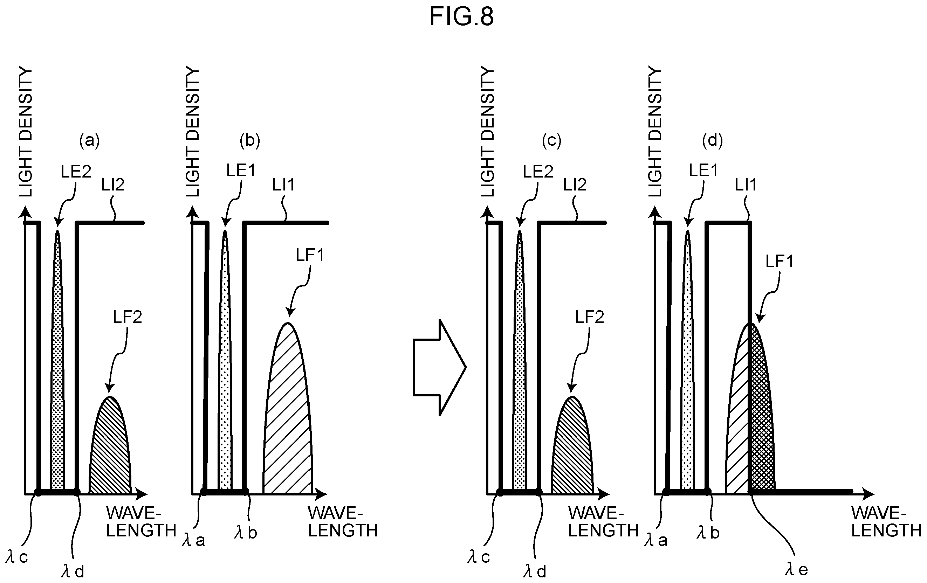

FIGS. 7 and 8 illustrate Variation 4 of the embodiment. Specifically, FIG. 7 illustrates a configuration of the imaging unit 51A according to Variation 4. Note that, FIG. 7 does not illustrate the lens unit 511 for convenience of description. Here, (a) of FIG. 8 and (c) of FIG. 8 illustrate the spectra of the second excitation light LE2 and the second fluorescent light LF2. Furthermore, (b) of FIG. 8 and (d) of FIG. 8 illustrate the spectra of the first excitation light LE1 and the first fluorescent light LF1. Then, lines LI1 indicate the transmission characteristics (filter characteristics) of the filter 11A. Furthermore, lines LI2 indicate the transmission characteristics (filter characteristics) of an excitation light cut filter 514. Note that, in FIG. 8, the horizontal axis represents a wavelength [nm], and the vertical axis represents a light density [W/nm].

FIG. 8 illustrates a case where the first fluorescent light LF1 has a light density larger than that of the second fluorescent light LF2 as in the above-described embodiment.

As illustrated in FIG. 7, the imaging unit 51A according to Variation 4 includes a prism 513, the excitation light cut filter 514, and the imaging element 512A in addition to the lens unit 511.

The prism 513 is disposed on the observation optical path P1. The prism 513 separates the beams of return light of the first and second beams of excitation light LE1 and LE2 into beams of light in two wavelength bands. The beams of return light propagate along the observation optical path P1, and pass through the lens unit 511. In Variation 4, the prism 513 separates the beams of return light into visible light and infrared light (invisible light). Furthermore, FIG. 7 illustrates a case where infrared light includes the first excitation light LE1 and the first fluorescent light LF1 and visible light includes the second excitation light LE2 and the second fluorescent light LF2.

The excitation light cut filter 514 cuts the second excitation light LE2 from visible light separated by the prism 513 after passing through the lens unit 511. Specifically, as indicated by the lines LI2 in (a) of FIG. 8 and (c) of FIG. 8, the excitation light cut filter 514 cuts light having the wavelengths λc to λd, which constitute a wavelength band of the second excitation light LE2. Note that, as indicated by the lines LI2 in (a) of FIG. 8 and (c) of FIG. 8, the excitation light cut filter 514 substantially or completely transmits all light outside the wavelength band of the wavelengths λc to λd.

The imaging element 512A according to Variation 4 includes first and second imaging elements 5121 and 5122. Note that not only two but three or more imaging elements may be provided as imaging elements 512A. Furthermore, types (e.g., sensitivity, size, and resolution) of these imaging elements may be the same as or different from each other.

The first imaging element 5121 includes a CCD or a CMOS, and images infrared light separated by the prism 513.

The second imaging element 5122 includes a CCD or a CMOS, and images the second fluorescent light LF2 that has passed through the excitation light cut filter 514 from visible light separated by the prism 513.

Here, as illustrated in FIG. 7, the filter 11A according to Variation 4 is disposed between the prism 513 and the first imaging element 5121. The filter 11A has a configuration in which a film of an inorganic material is optically formed on, for example, synthetic quartz. The synthetic quartz is a base material that emits a small amount of auto-fluorescent light. The filter 11A has a band-pass function, a short-pass function, or a long-pass function. Then, the filter 11A has excitation light cut function and a fluorescent light inhibiting function. In the excitation light cut function, the first excitation light LE1 contained in infrared light separated by the prism 513 is cut. In the fluorescent light inhibiting function, the first fluorescent light LF1 contained in return light is inhibited. Note that (b) of FIG. 8 illustrates a case where the filter 11A has only the excitation light cut function among the excitation light cut function and the fluorescent light inhibiting function. In contrast, (d) of FIG. 8 illustrates a case where the filter 11A has both the excitation light cut function and the fluorescent light inhibiting function.

Specifically, the excitation light cut function is as follows.

As indicated by the lines LI1 in (b) of FIG. 8 and (d) of FIG. 8, the filter 11A has the excitation light cut function of cutting light having wavelengths λa to λb and cutting light having wavelengths λc to λd. The wavelengths λa to λb constitute a wavelength band of the first excitation light LE1. The wavelengths λc to λd constitute a wavelength band of the second excitation light LE2.

Furthermore, the fluorescent light inhibiting function is as follows.

As indicated by a line LI1 in (d) of FIG. 8, the filter 11A has the fluorescent light inhibiting function of inhibiting light in a first wavelength band (wavelength λe or more), which is a part of a wavelength band of the first fluorescent light LF1.

Note that, as indicated by the line LI1 in (d) of FIG. 8, the filter 11A substantially or completely transmits all light outside the wavelength bands of the wavelengths λa to λb and the wavelength λe or more.

Then, when the filter 11A does not have the fluorescent light inhibiting function ((a) of FIG. 8 and (b) of FIG. 8), a signal value of the first output signal S1 from the first imaging element 5121 that has received the first fluorescent light LF1 is larger than a signal value of the second output signal S2 from the second imaging element 5122 that has received the second fluorescent light LF2. In contrast, when the filter 11A has the fluorescent light inhibiting function ((c) of FIG. 8 and (d) of FIG. 8), the signal value of the first output signal S1 approaches the signal value of the second output signal S2. Note that the signal value of the first output signal S1 is only required to approach the signal value of the second output signal S2. The signal values may be the same as each other, and are not required to be the same as each other. That is, the filter 11A inhibits light in the first wavelength band so that the signal value of the first output signal S1 approaches a predetermined signal value (signal value of second output signal S2) by the fluorescent light inhibiting function. The first wavelength band is at least a part of the wavelength band of the first fluorescent light LF1.

Even when the configuration of Variation 4 above is adopted, effects similar to those of the above-described embodiment are exhibited.

Note that, although Variation 4 above illustrates a case where infrared light includes the first excitation light LE1 and the first fluorescent light LF1 and visible light includes the second excitation light LE2 and the second fluorescent light LF2, this is not a limitation. The first and second beams of excitation light LE1 and LE2 and the first and second beams of fluorescent light LF1 and LF2 may be invisible light. Then, when the observation target OB is observed with visible light such as white light, an imaging element that images the visible light is required to be provided in addition to two imaging elements that image invisible light described above.

Furthermore, in Variation 4 above, the imaging unit 51A may function as a stereo camera. In the case, for example, the first imaging element 5121 that images the first fluorescent light LF1 functions as a left-eye imaging element. The second imaging element 5122 that images the second fluorescent light LF2 functions as a right-eye imaging element. Then, in a two-dimensional mode in which a two-dimensional image is displayed, a captured image captured by one of the first and second imaging elements 5121 and 5122 is displayed. In such a configuration, the filter according to the present disclosure is disposed on the optical path front stage side of at least one of the first and second imaging elements 5121 and 5122. The filter can correct the sensitivity difference between the first and second imaging elements 5121 and 5122 while effects similar to those of the above-described embodiment are exhibited.

### (Variation 5)

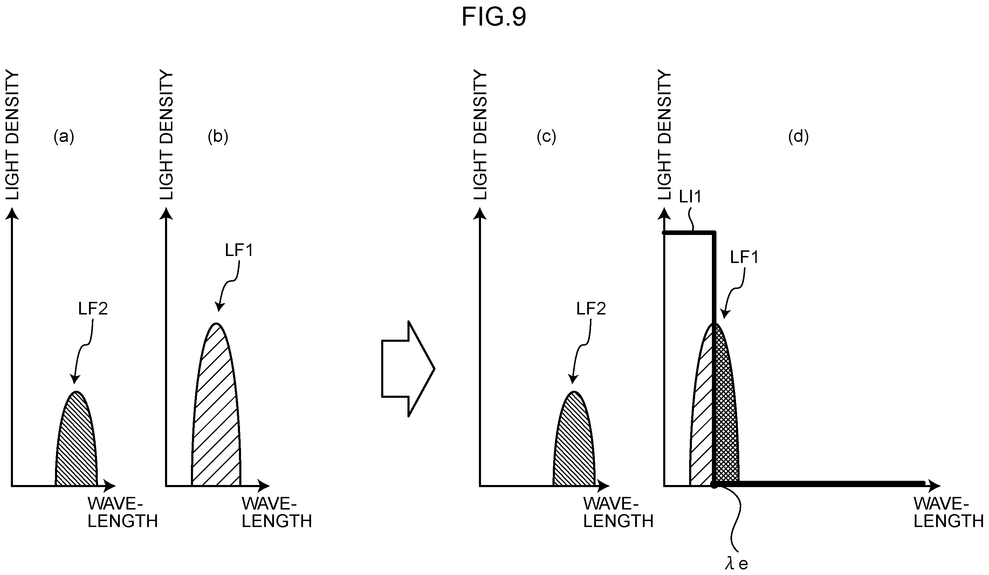

FIG. 9 illustrates Variation 5 of the embodiment. Specifically, FIG. 9 corresponds to FIG. 8. More specifically, (a) of FIG. 9 and (c) of FIG. 9 illustrate the spectrum of the second fluorescent light LF2 after the second fluorescent light LF2 has been transmitted through the excitation light cut filter 514. Furthermore, (b) of FIG. 9 illustrates the spectrum of the first fluorescent light LF1 immediately before the first fluorescent light LF1 is transmitted through the filter 11A according to Variation 5. Furthermore, (d) of FIG. 9 illustrates the spectrum of the first fluorescent light LF1 after the first fluorescent light LF1 has been transmitted through the filter 11A according to Variation 5.

Although, in Variation 4 above, the filter 11A has both the excitation light cut function and the fluorescent light inhibiting function, this is not a limitation. For example, as in the filter 11A according to Variation 5 indicated by a line LI1 in (d) of FIG. 9, a configuration having only the fluorescent light inhibiting function among the excitation light cut function and the fluorescent light inhibiting function may be adopted. That is, in Variation 5, a filter having the excitation light cut function is separately provided on the optical path front stage side of the filter 11A according to Variation 5.

Even when the filter 11A according to Variation 5 above is adopted, effects similar to those of Variation 4 above are exhibited.

### (Variation 6)

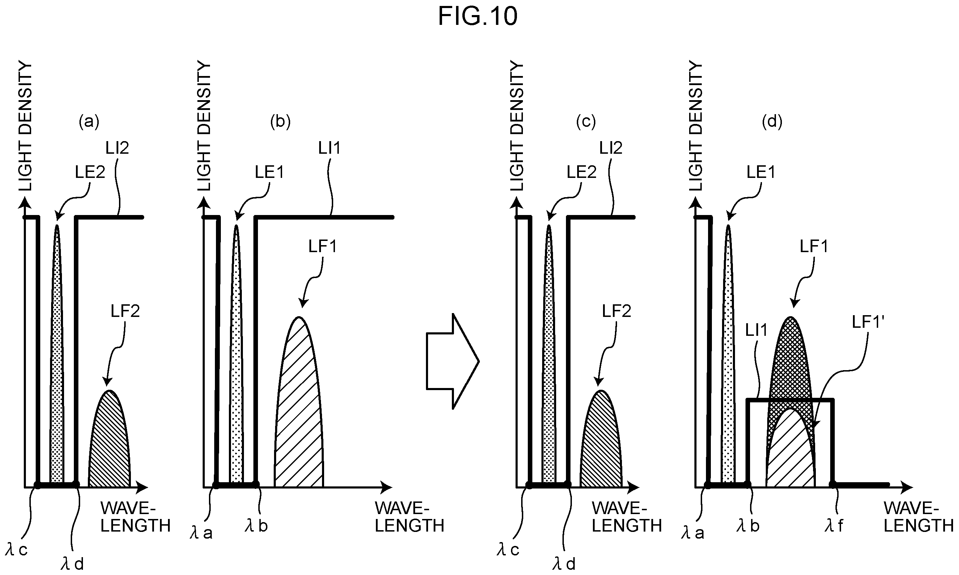

FIG. 10 illustrates Variation 6 of the embodiment. Specifically, FIG. 10 corresponds to FIG. 8.

In Variation 6, an ND filter is adopted as the filter 11A in Variation 4 above. Specifically, as indicated by a line LI1 in (d) of FIG. 10, the filter 11A according to Variation 6 has the excitation light cut function similar to that in Variation 4 above, and has the fluorescent light inhibiting function to be described below.

As indicated by the line LI1 in (d) of FIG. 10, the filter 11A according to Variation 6 has the fluorescent light inhibiting function of reducing light transmittance of a first wavelength band (wavelengths λb to λf) including all the wavelength band of the first fluorescent light LF1 and setting first fluorescent light LF1'. In the case, the hue of the first fluorescent light LF1' is maintained with respect to that of the first fluorescent light LF1. Note that a state in which the hue is maintained means, for example, a hue difference of approximately 5 or less in uniform color space such as L*a*b*.

Then, as in Variation 4 above, the filter 11A according to Variation 6 also makes the signal value of the first output signal S1 approach the signal value of the second output signal S2.

Even when the filter 11A according to Variation 6 above is adopted, effects similar to those of Variation 4 above are exhibited.

### (Variation 7)

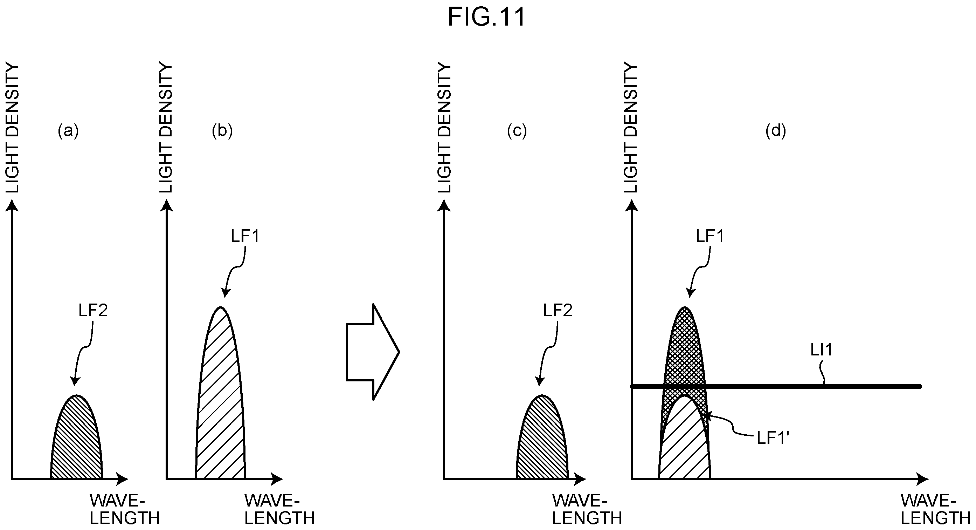

FIG. 11 illustrates Variation 7 of the embodiment. Specifically, FIG. 11 corresponds to FIG. 8. More specifically, (a) of FIG. 11 and (c) of FIG. 11 illustrate the spectrum of the second fluorescent light LF2 after the second fluorescent light LF2 has been transmitted through the excitation light cut filter 514. Furthermore, (b) of FIG. 11 illustrates the spectrum of the first fluorescent light LF1 immediately before the first fluorescent light LF1 is transmitted through the filter 11A according to Variation 7. Furthermore, (d) of FIG. 11 illustrates the spectrum of the first fluorescent light LF1 after the first fluorescent light LF1 has been transmitted through the filter 11A according to Variation 7.

Although, in Variation 6 above, the filter 11A has both the excitation light cut function and the fluorescent light inhibiting function, this is not a limitation. For example, as in the filter 11A according to Variation 7 indicated by a line LI1 in (d) of FIG. 11, a configuration having only the fluorescent light inhibiting function among the excitation light cut function and the fluorescent light inhibiting function may be adopted. That is, in Variation 7, a filter having the excitation light cut function is separately provided on the optical path front stage side of the filter 11A according to Variation 7.

Even when the filter 11A according to Variation 7 above is adopted, effects similar to those of Variation 6 above are exhibited.

### (Variation 8)

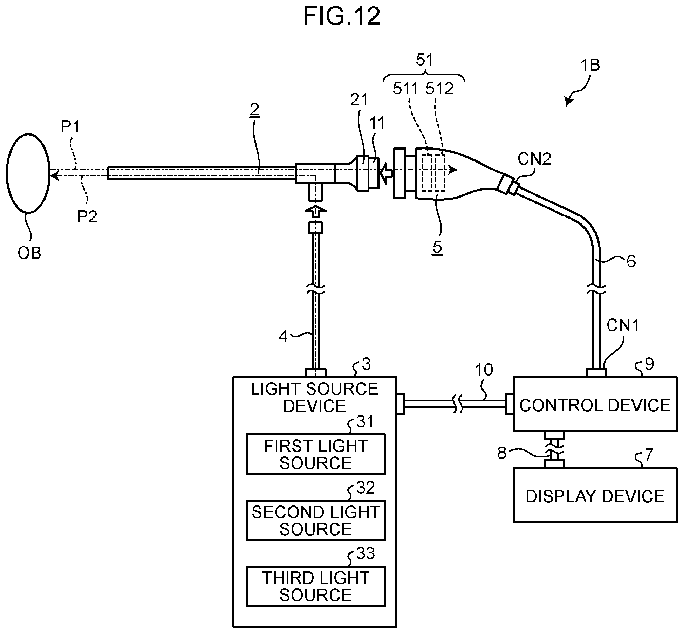

FIG. 12 illustrates Variation 8 of the embodiment. Specifically, FIG. 12 corresponds to FIG. 1.

The configuration in the above-described embodiment may be applied to surgery and the like using a plurality of medical agents in Table 1 below. Note that, in Table 1, "O " is attached to a medical agent to be used, and "×" is attached to a medical agent not to be used.

**Table 1**

| Category | Target disease | Medical agents | | | | |
|---|---|---|---|---|---|---|
| | | Fluorescein | ALM-488 | LUM-015 | 5-ALA | ICG |
| Surgery | Lung cancer | × | ○ | × | × | ○ |
| | Colorectal cancer | | | | | |
| | Childhood brain tumor | | | | | |
| | Breast cancer | | | ○ | | |
| Photo dynamic diagnosis | Brain tumor treatment | | | × | ○ | × |
| | Superficial cell cancer on urinary tract | ○ | × | | | |

Here, when excitation light in a wavelength band of approximately 470 to 480 [nm] is applied, fluorescein emits fluorescent light in a wavelength band of approximately 520 [nm]. Furthermore, when excitation light in a wavelength band of approximately 488 [nm] is applied, ALM-488 emits fluorescent light in a wavelength band of approximately 530 [nm]. Moreover, when excitation light in a wavelength band of approximately 650 [nm] is applied, LUM-015 emits fluorescent light L2 in a wavelength band of approximately 675 [nm]. Furthermore, when excitation light in a wavelength band of approximately 405 [nm] is applied, 5-ALA emits fluorescent light in a wavelength band of approximately 530 to 630 [nm]. Moreover, when excitation light in a wavelength band of approximately 805 [nm] is applied, ICG emits the fluorescent light L2 in a wavelength band of approximately 830 [nm].

For example, when lymph node dissection is performed in lobectomy for treating lung cancer, ALM-488 and ICG are used as illustrated in Table 1. That is, ALM-488 visualizes a left recurrent laryngeal nerve. ICG visualizes a lymph node. The left recurrent laryngeal nerve and the lymph node are identified by visualizing the left recurrent laryngeal nerve and the lymph node as described above, so that recurrent laryngeal nerve paralysis in the lobectomy can be reduced. In the case, one of two medical agents of ALM-488 and ICG is excited by one of the first and second beams of excitation light. Furthermore, the other of the two medical agents of ALM-488 and ICG is excited by the other of the first and second beams of excitation light. Note that the filter 11 transmits fluorescent light (corresponding to second fluorescent light according to present disclosure) from one medical agent having weak fluorescent light of the two medical agents of ALM-488 and ICG. Furthermore, for fluorescent light (corresponding to first fluorescent light according to present disclosure) from the other medical agent (corresponding to first medical agent according to present disclosure), the filter 11 inhibits at least a part of a wavelength band of the fluorescent light so that an output signal value from the imaging element 512 that has received light approaches a predetermined signal value. Then, a doctor who performs the lobectomy can grasp the positions of the left recurrent laryngeal nerve and the lymph node from an image displayed on the display device 7.

Furthermore, for example, when lateral side dissection is performed in rectectomy for treating colorectal cancer, ALM-488 and ICG are used as illustrated in Table 1. That is, ALM-488 visualizes a neurovascular bundle. ICG visualizes a lymph node. The neurovascular bundle and the lymph node are identified by visualizing the neurovascular bundle and the lymph node as described above, so that urinary disturbance, defecation disturbance, male function disturbance, or motor function disturbance due to nerve damage in the rectectomy can be reduced. In the case, one of two medical agents of ALM-488 and ICG is excited by one of the first and second beams of excitation light. Furthermore, the other of the two medical agents of ALM-488 and ICG is excited by the other of the first and second beams of excitation light. Note that the filter 11 transmits fluorescent light (corresponding to second fluorescent light according to present disclosure) from one medical agent having weak fluorescent light of the two medical agents of ALM-488 and ICG. Furthermore, for fluorescent light (corresponding to first fluorescent light according to present disclosure) from the other medical agent (corresponding to first medical agent according to present disclosure), the filter 11 inhibits at least a part of a wavelength band of the fluorescent light so that an output signal value from the imaging element 512 that has received light approaches a predetermined signal value. Then, a doctor who performs the rectectomy can grasp the positions of the neurovascular bundle and the lymph node from an image displayed on the display device 7.

Moreover, for example, when malignant tumor is excised in malignant tumor excision for treating a childhood brain tumor, ALM-488 and ICG are used as illustrated in Table 1. That is, ALM-488 visualizes a nerve. ICG visualizes a blood vessel (bloodstream). The malignant tumor, the nerve, and the blood vessel are identified by visualizing the nerve and the blood vessel as described above, so that cancer recurrence after the malignant tumor is excised can be prevented, and damage of the nerve and the blood vessel in the malignant tumor excision can be prevented. In the case, one of two medical agents of ALM-488 and ICG is excited by one of the first and second beams of excitation light. Furthermore, the other of the two medical agents of ALM-488 and ICG is excited by the other of the first and second beams of excitation light. Note that the filter 11 transmits fluorescent light (corresponding to second fluorescent light according to present disclosure) from one medical agent having weak fluorescent light of the two medical agents of ALM-488 and ICG. Furthermore, for fluorescent light (corresponding to first fluorescent light according to present disclosure) from the other medical agent (corresponding to first medical agent according to present disclosure), the filter 11 inhibits at least a part of a wavelength band of the fluorescent light so that an output signal value from the imaging element 512 that has received light approaches a predetermined signal value. Then, a doctor who performs the malignant tumor excision can grasp the positions of the nerve and the blood vessel from an image displayed on the display device 7.

Furthermore, for example, when malignant tumor excision and axillary lymph node dissection are performed in mastectomy for treating breast cancer, ALM-488, LUM-015, and ICG are used as illustrated in Table 1. That is, ALM-488 visualizes a nerve such as an intercostobrachial nerve and a thoracodorsal nerve. LUM-015 visualizes the malignant tumor. ICG visualizes a lymph node. The malignant tumor, the nerve, and the lymph node are identified by visualizing the nerve, the malignant tumor, and the lymph node, so that damage of the nerve in the mastectomy can be reduced. In the case, since three medical agents of ALM-488, LUM-015, and ICG are used, a configuration of a medical observation system 1B as illustrated in FIG. 12 is required. The medical observation system 1B includes first to third light sources 31 to 33 that emit beams of excitation light for exciting the three medical agents. Note that the filter 11 transmits fluorescent light (corresponding to second fluorescent light according to present disclosure) from at least one medical agent having weak fluorescent light of the three medical agents of ALM-488, LUM-015, and ICG. Furthermore, for fluorescent light (corresponding to first fluorescent light according to present disclosure) from the other medical agent (corresponding to first medical agent according to present disclosure), the filter 11 inhibits at least a part of a wavelength band of the fluorescent light so that an output signal value from the imaging element 512 that has received light approaches a predetermined signal value. Then, a doctor who performs the mastectomy can grasp the positions of the nerve, the malignant tumor, and the lymph node from an image displayed on the display device 7.

Moreover, for example, when a brain tumor is treated by using photo dynamic diagnosis (PDD), ALM-488 and 5-aminolevulinic acid (5-ALA) are used as illustrated in Table 1. That is, fluorescein, which is a fluorescent dye of ALM-488, visualizes the vicinity of a tumor. Protoporphyrin (PpIX) biosynthesized from 5-ALA visualizes tumor cells. Damage in treating a brain tumor can be prevented and the tumor can be safely removed by visualizing tumor cells including the vicinity of the tumor. In the case, one of two medical agents of ALM-488 and 5-ALA is excited by one of the first and second beams of excitation light. Furthermore, the other of the two medical agents of ALM-488 and 5-ALA is excited by the other of the first and second beams of excitation light. Note that the filter 11 transmits fluorescent light (corresponding to second fluorescent light according to present disclosure) from 5-ALA having weak fluorescent light of the two medical agents of ALM-488 and 5-ALA. Furthermore, for fluorescent light (corresponding to first fluorescent light according to present disclosure) from ALM-488 (corresponding to first medical agent according to present disclosure), the filter 11 inhibits at least a part of a wavelength band of the fluorescent light so that an output signal value from the imaging element 512 that has received light approaches a predetermined signal value. Then, a doctor who treats a brain tumor can grasp the positions of the tumor cell and the vicinity of the tumor from an image displayed on the display device 7.

Furthermore, for example, when transurethral cystectomy is performed in surgically treating superficial cell cancer on a urinary tract by using the photo dynamic diagnosis, fluorescein and 5-ALA are used as illustrated in Table 1. That is, PpIX biosynthesized from 5-ALA visualizes the tumor. Here, PpIX has a disadvantage of visualizing not only a tumor but highly metabolic inflamed tissues in the bladder. Therefore, fluorescein reduces false positives of the highly metabolic inflamed tissues caused by PpIX. In the case, one of two medical agents of fluorescein and 5-ALA is excited by one of the first and second beams of excitation light. Furthermore, the other of the two medical agents of fluorescein and 5-ALA is excited by the other of the first and second beams of excitation light. Note that the filter 11 transmits fluorescent light (corresponding to second fluorescent light according to present disclosure) from 5-ALA having weak fluorescent light of the two medical agents of fluorescein and 5-ALA. Furthermore, for fluorescent light (corresponding to first fluorescent light according to present disclosure) from fluorescein (corresponding to first medical agent according to present disclosure), the filter 11 inhibits at least a part of a wavelength band of the fluorescent light so that an output signal value from the imaging element 512 that has received light approaches a predetermined signal value. Then, a doctor who performs the transurethral cystectomy can grasp the position of the tumor and the false positives of the highly metabolic inflamed tissues caused by PpIX from an image displayed on the display device 7.

Even when the configuration of Variation 8 above is adopted, effects similar to those of the above-described embodiment are exhibited.

### (Variation 9)

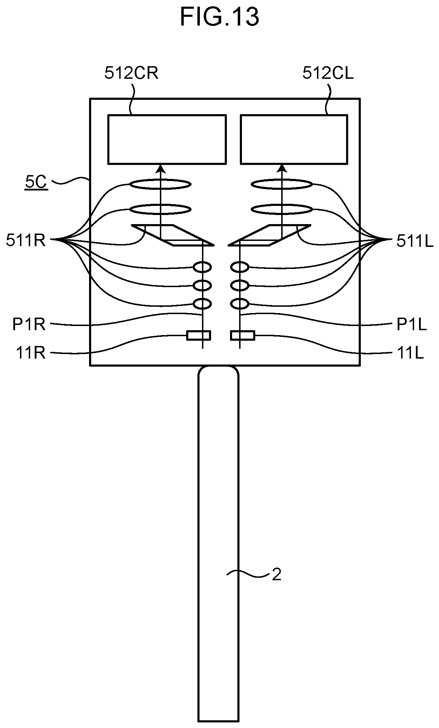

FIG. 13 illustrates Variation 9 of the embodiment.

In the above-described embodiment, the camera head 5 may function as a stereo camera. For convenience of description, the camera head 5 according to Variation 9 will be hereinafter referred to as a camera head 5C. That is, the camera head 5C images right-eye observation light (right-eye subject image) and left-eye observation light (left-eye subject image), which have parallax with each other.

Here, in addition to a common scope (scope having one optical path), a scope of a twin-lens relay method or a single-lens pupil division method to be described below may be adopted as the insertion portion 2.

In a scope of the twin-lens relay method, two optical paths are arranged in parallel in the scope. Furthermore, optical systems are arranged in the two optical paths. Then, the scope of a twin-lens relay method captures and emits right-eye observation light and left-eye observation light, which have parallax with each other, with the two optical systems (e.g., see JP H6-160731 A).

Furthermore, in a scope of the single-lens pupil division method, one optical path is provided in the scope. Furthermore, an optical system is arranged in the one optical path. Moreover, a pupil division unit is provided at a pupil position of the optical system. The pupil division unit divides a light flux in a pupil into two regions. Then, the scope of a single-lens pupil division method captures observation light with the optical system, divides the observation light into right-eye observation light and left-eye observation light, which have parallax with each other, with the pupil division unit, and emits the divided beams of observation light (e.g., see JP H6-59199 A).

As illustrated in FIG. 13, the camera head 5C includes a right-eye lens unit 511R, a right-eye imaging module 512CR, a left-eye lens unit 511L, and a left-eye imaging module 512CL.

As illustrated in FIG. 13, each of the right-eye lens unit 511R and the right-eye imaging module 512CR is arranged on an observation optical path P1R through which right-eye observation light propagates.

As illustrated in FIG. 13, the right-eye lens unit 511R includes a plurality of lenses and prisms, captures a right-eye subject image via the insertion portion 2, and guides the right-eye subject image to the right-eye imaging module 512CR.

The right-eye imaging module 512CR includes at least one imaging element including a CCD or a CMOS, and images the right-eye subject image guided by the right-eye lens unit 511R.

As illustrated in FIG. 13, each of the left-eye lens unit 511L and the left-eye imaging module 512CL is arranged on an observation optical path P1L through which left-eye observation light propagates.

As illustrated in FIG. 13, the left-eye lens unit 511L includes a plurality of lenses and prisms, captures a left-eye subject image via the insertion portion 2, and guides the left-eye subject image to the left-eye imaging module 512CL.

The left-eye imaging module 512CL includes at least one imaging element including a CCD or a CMOS, and captures the right-eye subject image guided by the left-eye lens unit 511L.

Note that, in Variation 9, the right-eye and left-eye imaging modules 512CR and 512CL are different from each other. Specifically, the right-eye and left-eye imaging modules 512CR and 512CL have at least partially different imageable wavelength bands. For example, the right-eye imaging module 512CR can image fluorescent light (corresponding to first fluorescent light according to present disclosure) from a medical agent (corresponding to first medical agent according to present disclosure) that emits non-weak fluorescent light. In contrast, the left-eye imaging module 512CL can image fluorescent light (corresponding to second fluorescent light according to present disclosure) from a medical agent that emits weak fluorescent light. Then, in a two-dimensional mode in which a two-dimensional image is displayed, a captured image captured by one of the right-eye and left-eye imaging modules 512CR and 512CL is displayed. In such a configuration, as illustrated in FIG. 13, a filter 11R for non-weak fluorescent light is arranged on the observation optical path P1R through which right-eye observation light propagates, and a filter 11L for weak fluorescent light is arranged on the observation optical path P1L through which left-eye observation light propagates. For example, when an OD value in a wavelength band of non-weak fluorescent light in the filter 11R is compared with an OD value in a wavelength band of weak fluorescent light in the filter 11L, the former OD value is set high. That is, the filter 11R inhibits at least a part of a wavelength band of non-weak fluorescent light so that an output signal value from the right-eye imaging module 512CR that has received light approaches a predetermined signal value.

Even when the configuration of Variation 9 above is adopted, effects similar to those of the above-described embodiment are exhibited.

### (Variation 10)

The medical observation system according to Variation 10 uses a so-called video scope (flexible endoscope) including an imaging unit on the distal end side of the insertion portion. For convenience of description, the medical observation system 1 according to Variation 10 will be hereinafter referred to as a medical observation system 1D.

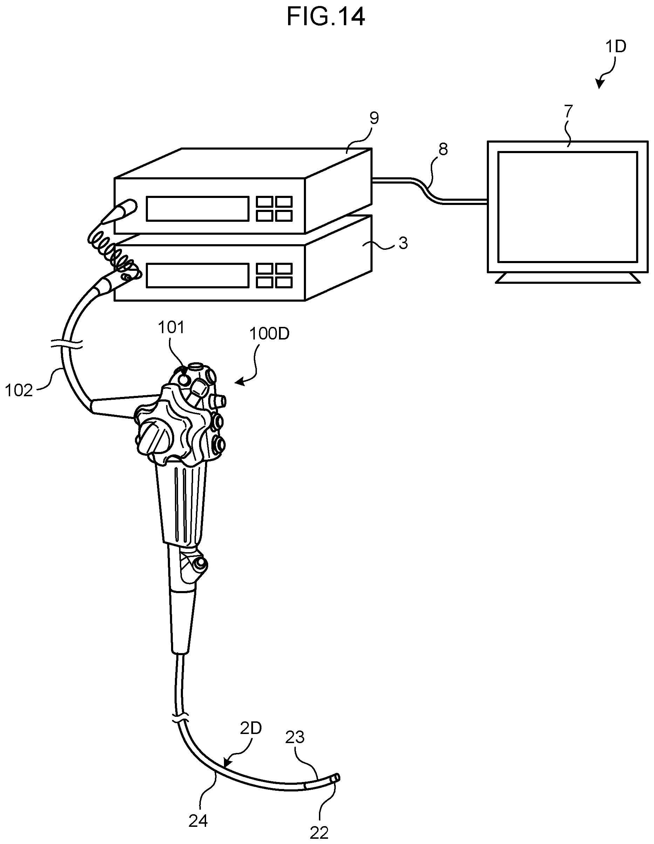

FIG. 14 illustrates Variation 10 of the embodiment.

As illustrated in FIG. 14, the medical observation system 1D includes an endoscope 100D, the light source device 3, the control device 9, and the display device 7. The endoscope 100D captures an in-vivo image of an observed region by inserting an insertion portion 2D into a living body, and outputs the captured image. The light source device 3 emits the first and second beams of excitation light LE1 and LE2 from a distal end of the endoscope 100D. The control device 9 processes the captured image output from the endoscope 100D. The display device 7 is connected to the control device 9 via the second transmission cable 8, and displays an image based on a video signal processed by the control device 9.

As illustrated in FIG. 14, the endoscope 100D includes the insertion portion 2D, an operating unit 101, and a universal cord 102. The insertion portion 2D has flexibility and an elongated shape. The operating unit 101 is connected to a proximal end side of the insertion portion 2D, and receives various operations. The universal cord 102 extends from the operating unit 101 in a direction different from the direction in which the insertion portion 2D extends, and incorporates various cables to be connected to the light source device 3 and the control device 9.

As illustrated in FIG. 14, the insertion portion 2D includes a distal end portion 22, a bent portion 23, and a flexible tube portion 24. The bent portion 23 is connected to the proximal end side of the distal end portion 22, includes a plurality of bent pieces, and can be freely bent. The flexible tube portion 24 is connected to the proximal end side of the bent portion 23, and has flexibility and a long shape.

Although not specifically illustrated, the distal end portion 22 incorporates a configuration substantially similar to the camera head 5 described above in the embodiment. Then, the captured image obtained by the distal end portion 22 (imaging element) is output to the control device 9 via the operating unit 101 and the universal cord 102.

Furthermore, although not specifically illustrated, in the medical observation system 1D, a filter similar to the filter 11 described above in the embodiment is disposed on an observation optical path through which the beams of return light of the first and second beams of excitation light LE1 and LE2 propagate from the observation target to the imaging element.

Even when the configuration of Variation 10 above is adopted, effects similar to those of the above-described embodiment are exhibited.

### (Variation 11)

The medical observation system according to Variation 11 uses a surgical microscope that enlarges and images a predetermined visual field region inside a subject (living body) or on the surface of the subject (surface of living body) to be observed. For convenience of description, the medical observation system 1 according to Variation 11 will be hereinafter referred to as a medical observation system 1E.

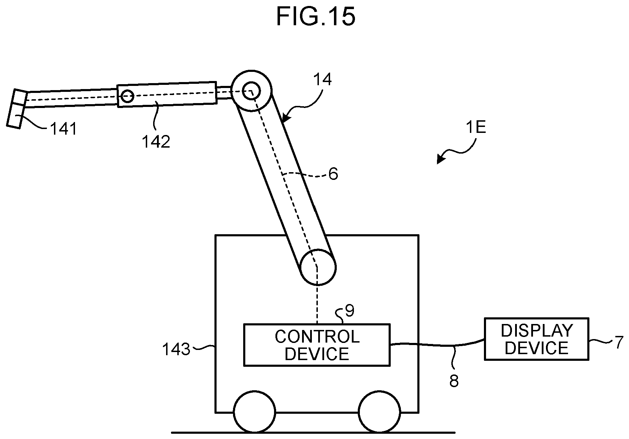

FIG. 15 illustrates Variation 11 of the embodiment.

As illustrated in FIG. 15, the medical observation system 1E includes a surgical microscope 14, the control device 9, and the display device 7. The surgical microscope 14 captures an image for observing a subject, and outputs the captured image. The control device 9 processes the captured image output from the surgical microscope 14. The display device 7 is connected to the control device 9 via the second transmission cable 8, and displays an image based on a video signal processed by the control device 9.

As illustrated in FIG. 15, the surgical microscope 14 includes a microscope portion 141, a support portion 142, and a base portion 143. The microscope portion 141 enlarges and images a minute part of the subject, and outputs the captured image. The support portion 142 is connected to a proximal end portion of the microscope portion 141, and includes an arm that rotatably supports the microscope portion 141. The base portion 143 rotatably holds a proximal end portion of the support portion 142, and can move on a floor surface.

Then, as illustrated in FIG. 15, the control device 9 is installed in the base portion 143. Furthermore, although not specifically illustrated, the light source device 3 is also installed in the base portion 143. The light source device 3 emits the first and second beams of excitation light LE1 and LE2 from the surgical microscope 14 to the observation target.

Note that the base portion 143 may be fixed to a ceiling, a wall surface, or the like to support the support portion 142 instead of being movably provided on the floor surface.

Although not specifically illustrated, the microscope portion 141 incorporates a configuration substantially similar to the camera head 5 described above in the embodiment. Then, the captured image obtained by the microscope portion 141 (imaging element) is output to the control device 9 via the first transmission cable 6 arranged along the support portion 142.

Furthermore, although not specifically illustrated, in the medical observation system 1E, a filter similar to the filter 11 described above in the embodiment is disposed on an observation optical path through which the beams of return light of the first and second beams of excitation light LE1 and LE2 propagate from the observation target to the imaging element.

Even when the configuration of Variation 11 above is adopted, effects similar to those of the above-described embodiment are exhibited.

### (Variation 12)

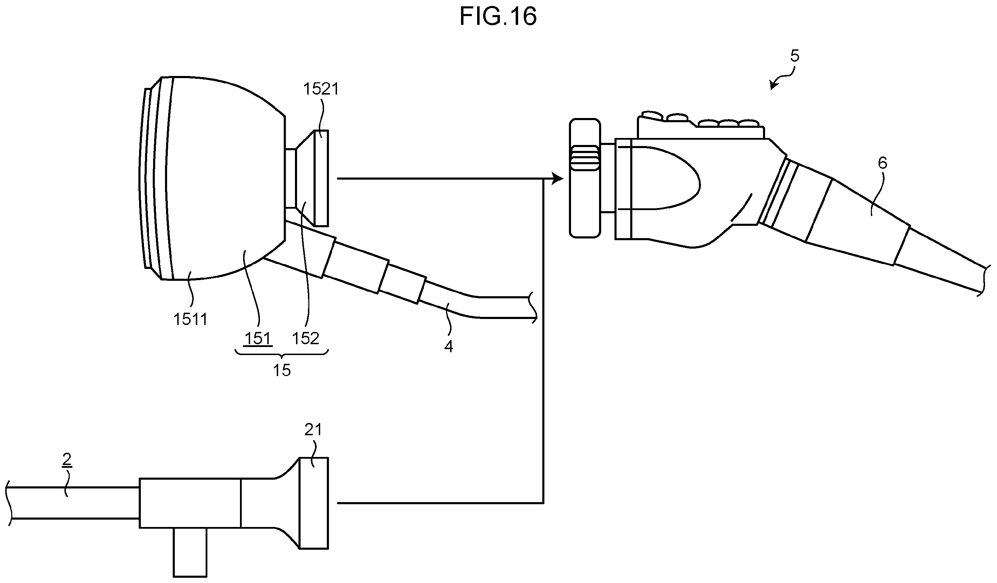

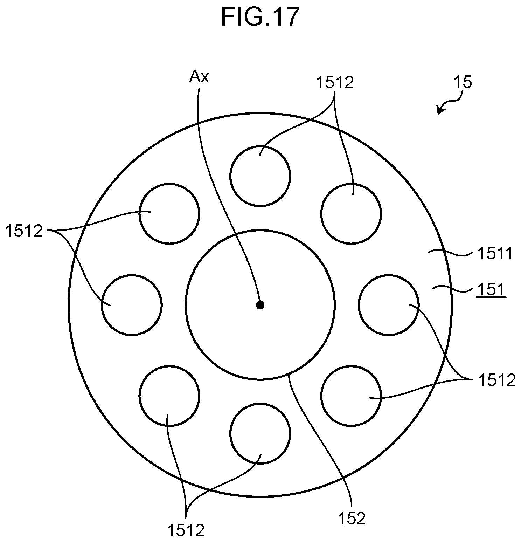

FIGS. 16 and 17 illustrate Variation 12 of the embodiment. Specifically, FIG. 16 illustrates a ring light 15 viewed from a lateral side. FIG. 17 illustrates the ring light 15 viewed from a front side (left side in FIG. 16).

In Variation 12, in addition to the insertion portion 2 described above in the embodiment, the ring light 15 in FIGS. 16 and 17 is detachably connected to the camera head 5. That is, as illustrated in FIG. 16, the insertion portion 2 or the ring light 15 may be connected to the camera head 5 depending on a state of use of a user.

The ring light 15 is not inserted into the observation target OB like the insertion portion 2. The ring light 15 supplies the first and second beams of excitation light LE1 and LE2 to a surgical portion, and captures the beams of return light of the first and second beams of excitation light LE1 and LE2 from the surgical portion. As illustrated in FIGS. 16 and 17, the ring light 15 includes an illumination unit 151 and a subject image capturing unit 152 that captures a subject image.

As illustrated in FIGS. 16 and 17, the illumination unit 151 includes a casing 1511 and a plurality of illumination lenses 1512.

The casing 1511 has an annular shape centered on an optical axis Ax. Then, the other end of the light guide 4 is detachably connected to the casing 1511.

As illustrated in FIG. 17, the plurality of illumination lenses 1512 is arranged at predetermined intervals along the peripheral direction centered on the optical axis Ax on the end surface on the front side of the casing 1511. Then, the plurality of illumination lenses 1512 applies, to the surgical portion, the first and second beams of excitation light LE1 and LE2, which have been supplied from the light source device 3 and introduced into the casing 1511 via the light guide 4.

The subject image capturing unit 152 extends along the optical axis Ax. Furthermore, an optical system is provided in the subject image capturing unit 152. The optical system includes one or a plurality of lenses, and collects the beams of return light of the first and second beams of excitation light LE1 and LE2 applied from the plurality of illumination lenses 1512 via the surgical portion. Moreover, a connection portion 1521 is provided at an end on the proximal end side (right side in FIG. 16) of the subject image capturing unit 152. The connection portion 1521 is designed (has shape) so as to be compatible with the eyepiece portion 21 of the insertion portion 2, and detachably connected to the camera head 5.

Then, although not specifically illustrated, in Variation 12, a filter similar to the filter 11 described above in the embodiment is disposed on an observation optical path through which the beams of return light of the first and second beams of excitation light LE1 and LE2 propagate from the observation target to the imaging element. More specifically, the filter is provided at a front surface portion (front surface on optical path front stage side) of the subject image capturing unit 152.

Even when the configuration of Variation 12 above is adopted, effects similar to those of the above-described embodiment are exhibited.

Note that the following configurations also belong to the technical scope of the present disclosure.

(22) A camera head including an imaging element that images first fluorescent light emitted from a substance contained in an observation target by applying first excitation light, the camera head including a filter that is disposed on an observation optical path through which the first fluorescent light propagates and that inhibits light in a first wavelength band, which is at least a part of a wavelength band of the first fluorescent light, so that a first output signal value from the imaging element approaches a predetermined signal value.

### Reference Signs List

- 1, 1B, 1D, 1E: MEDICAL OBSERVATION SYSTEM
- 2, 2D: INSERTION PORTION
- 3: LIGHT SOURCE DEVICE
- 4: LIGHT GUIDE
- 5, 5C: CAMERA HEAD
- 6: FIRST TRANSMISSION CABLE
- 7: DISPLAY DEVICE
- 8: SECOND TRANSMISSION CABLE
- 9: CONTROL DEVICE
- 10: THIRD TRANSMISSION CABLE
- 11, 11A: FILTER
- 14: SURGICAL MICROSCOPE
- 15: RING LIGHT
- 21: EYEPIECE PORTION
- 22: DISTAL END PORTION
- 23: BENT PORTION
- 24: FLEXIBLE TUBE PORTION
- 31: FIRST LIGHT SOURCE
- 32: SECOND LIGHT SOURCE
- 33: THIRD LIGHT SOURCE
- 51, 51A: IMAGING UNIT
- 100D: ENDOSCOPE
- 101: OPERATING UNIT
- 102: UNIVERSAL CORD
- 141: MICROSCOPE PORTION
- 142: SUPPORT PORTION
- 143: BASE PORTION
- 151: ILLUMINATION UNIT
- 152: SUBJECT IMAGE CAPTURING UNIT
- 511: LENS UNIT
- 511L: LEFT-EYE LENS UNIT
- 512R: RIGHT-EYE LENS UNIT
- 512, 512A: IMAGING ELEMENT
- 512CL: LEFT-EYE IMAGING MODULE
- 512CR: RIGHT-EYE IMAGING MODULE
- 513: PRISM
- 514: EXCITATION LIGHT CUT FILTER
- 1511: CASING
- 1512: ILLUMINATION LENS
- 1521: CONNECTION PORTION
- 5121: FIRST IMAGING ELEMENT
- 5122: SECOND IMAGING ELEMENT
- Ax: OPTICAL AXIS
- LI, LI1, LI2: LINE
- LE1: FIRST EXCITATION LIGHT
- LE2: SECOND EXCITATION LIGHT
- LF1, LF1': FIRST FLUORESCENT LIGHT
- LF2: SECOND FLUORESCENT LIGHT
- OB: OBSERVATION TARGET
- P1, P1L, P1R: OBSERVATION OPTICAL PATH
- P2: OPTICAL PATH

## Claims

1. A medical observation system comprising:
a light source device configured to emit first excitation light;
an imaging element configured to image first fluorescent light emitted from a substance contained in an observation target by applying the first excitation light; and
a filter disposed on an observation optical path through which the first fluorescent light propagates from the observation target to the imaging element and configured to inhibit light in a first wavelength band, which is at least a part of a wavelength band of the first fluorescent light, such that a first output signal value from the imaging element that has received the first fluorescent light approaches a predetermined signal value.

2. The medical observation system according to claim 1, further comprising an insertion portion including a part of the observation optical path and configured to guide the first fluorescent light to the imaging element,
wherein the filter is disposed on the observation optical path of the insertion portion.

3. The medical observation system according to claim 1, further comprising a camera head including the imaging element,
wherein the filter is disposed on the observation optical path of the camera head.

4. The medical observation system according to claim 1, further comprising:
an insertion portion including a part of the observation optical path and configured to guide the first fluorescent light to the imaging element; and
a camera head including the imaging element,
wherein the filter is disposed between an eyepiece portion of the optical visual tube and the camera head on the observation optical path.

5. The medical observation system according to claim 1, wherein the filter is detachable from a member constituting the observation optical path.

6. The medical observation system according to claim 1, wherein the filter is configured to adjust light transmittance of the first wavelength band.

7. The medical observation system according to claim 1, wherein the filter is a long-pass filter or a short-pass filter.

8. The medical observation system according to claim 1, wherein the filter is an ND filter.

9. The medical observation system according to claim 1, wherein the filter has an excitation light cut function of partially, substantially, or completely inhibiting the first excitation light.

10. The medical observation system according to claim 1, wherein the first fluorescent light is emitted from a first medical agent administered to the observation target.

11. The medical observation system according to claim 1, wherein the predetermined signal value is smaller than a signal value at which a pixel of the imaging element saturates.

12. The medical observation system according to claim 1,
wherein the light source device is configured to emit each of the first excitation light and second excitation light,
the imaging element is configured to image each of the first fluorescent light and second fluorescent light emitted from a substance contained in the observation target by applying the second excitation light, and
the predetermined signal value is a second output signal value from the imaging element that has received the second fluorescent light.

13. The medical observation system according to claim 1,
wherein the light source device is configured to emit each of the first excitation light and second excitation light,
the imaging element includes:
a first imaging element configured to image the first fluorescent light; and
a second imaging element configured to image second fluorescent light emitted from a substance contained in the observation target by applying the second excitation light,
the first output signal value is provided from the first imaging element that has received the first fluorescent light, and
the predetermined signal value is a second output signal value from the second imaging element that has received the second fluorescent light.

14. The medical observation system according to claim 1, wherein the predetermined signal value is set based on brightness-related information for specifying brightness in performing fluorescent light observation.

15. The medical observation system according to claim 14, wherein the brightness-related information relates to at least one or a plurality of a type of the observation target, a type of a medical agent, a surgical procedure, and a device constituting a system.

16. The medical observation system according to claim 1, wherein only one imaging element is provided.

17. The medical observation system according to claim 1, wherein a plurality of imaging elements is provided.

18. The medical observation system according to claim 1, wherein
the light source device includes a first light source configured to emit the first excitation light, and
one or a plurality of first light sources is provided.

19. The medical observation system according to claim 1, wherein
the light source device includes a first light source configured to emit the first excitation light, and
the first light source includes an LED or a semiconductor laser.

20. A filter disposed on an observation optical path through which the first fluorescent light propagates from an observation target to which first excitation light is applied to an imaging element that images first fluorescent light emitted from a substance contained in the observation target by applying the first excitation light and is configured to inhibit light in a first wavelength band, which is at least a part of a wavelength band of the first fluorescent light, such that a first output signal value from the imaging element that has received the first fluorescent light approaches a predetermined signal value.

21. An optical visual tube for applying first excitation light to an observation target and capturing first fluorescent light emitted from a substance contained in the observation target by applying the first excitation light, the optical visual tube comprising
a filter disposed on an observation optical path through which the first fluorescent light propagates and configured to inhibit light in a first wavelength band, which is at least a part of a wavelength band of the first fluorescent light, such that a first output signal value from an imaging element that has received the first fluorescent light approaches a predetermined signal value.
